# EUROPÄISCHE PATENTSCHRIFT

(11) **EP 2 811 216 B1**
(45) Veröffentlichungstag und Bekanntmachung des Hinweises auf die Patenterteilung: **11.10.2017**
(21) Anmeldenummer: 14154783.6
(22) Anmeldetag: 12.02.2014
(51) Int. Cl.: F16P 1/06

(54) **Laserschutzmaterial und Laserschutzkomponente**
Laser protection material and laser protection components
Matériau de protection laser et composant de protection laser

(30) Priorität: 26.02.2013 DE 102013203120
(43) Veröffentlichungstag der Anmeldung: 10.12.2014
(73) Patentinhaber: Laservision GmbH & Co. KG, 90766 Fürth (DE)
(72) Erfinder: Fröhlich, Thomas, 91207 Lauf an der Pegnitz (DE)
(74) Vertreter: Rau, Schneck & Hübner Patentanwälte Rechtsanwälte PartGmbB

(56) Entgegenhaltungen:
- DE-A1-102010 003 366
- US-A- 3 853 783

## Beschreibung

Die Erfindung betrifft ein Laserschutzmaterial für ein Laserschutzfilter mit einem wirksamen Wellenlängenbereich sowie eine Laserschutzkomponente aus einem solchen Laserschutzmaterial.

Es sind bereits verschiedene Laserschutzmaterialien bekannt. Aus diesen Materialien werden unter anderem auch Schutzfilter zu einem Einsatz in Laserschutzbrillen und/oder in Laserschutzscheiben hergestellt. Diese Filter dienen dem Schutz vor Strahlung von Diodenlasern, Festkörperlasern, Scheibenlasern, Faserlasern und Titansaphir-Lasern. Derzeit kommt als Kunststoffmaterial für solche Schutzfilter üblicherweise Polycarbonat zum Einsatz. Die Filterwirkung liegt im nahen Infrarot-Bereich bei Wellenlängen zwischen 750 nm und 1150 nm. Mit derartigen Polycarbonat-basierten Schutzfiltern lassen sich Laserschutzstufen gemäß EN 207:2009 von bis zu D LB6 erreichen. Eine maximale Laserschutzstufe D LB6 ist aber insbesondere bei der Laser-Materialbearbeitung oftmals nicht ausreichend.

In der DE 10 2005 009 613 A1 wird ein als ein polymeres Verbundmaterial ausgebildetes Laserschutzmaterial beschrieben, welches bei auftreffender Laserstrahlung mit einer mit der Energieaufnahme verbundenen Strukturumwandlung reagiert.

In der US 2009/0204186 A1 und der EP 0 992 832 A1 werden jeweils Brillengläser beschrieben, wobei das Material dieser Brillengläser ein im sichtbaren Wellenlängenbereich transparentes Matrixmaterial aus einem Kunststoff, insbesondere Polyurethan, und ein homogen in das Matrixmaterial eingebettetes Absorbermaterial, insbesondere einen UV-Blocker, umfasst.

In der DE 10 2006 003 450 A1 und der WO 94/15557 A2 wird jeweils eine Laserschutzvorrichtung beschrieben, bei der ein Material aus vollständig aufgeschäumten Schaum-Schichten zum Einsatz kommt.

In der DE 20 2006 020 429 U1 wird ein Verfahren zur Herstellung eines mit einer glänzenden transparenten Kunststoffbeschichtung versehenen Zierteils zur Verwendung in einem Kraftfahrzeug beschrieben. Bei der transparenten Kunststoffbeschichtung handelt es sich um eine Polyurethan-Beschichtung, die durch Einspritzen einer Polyol-Komponente und einer Isocyanat-Komponente in eine Spritzgussform hergestellt wird, wobei zur Erzielung des glänzenden Effekts der Polyol-Komponente ein Mattierungsmittel, beispielsweise Kieselsäure, zugesetzt wird.

In der DE 10 2010 003 366 A1 wird ein Kunststoff-Matrixmaterial beschrieben, das sich zum Kunststoffschweißen und zum Markieren bzw. Beschriften jeweils unter anderem mittels einer Laserstrahlung eignet. Das Kunststoff-Matrixmaterial umfasst ein in dem Matrixmaterial eingebettetes Absorbermaterial, welches Laserlicht absorbiert und eine lokale Erwärmung in dem Matrixmaterial am Ort der Bestrahlung bewirkt, sowie ein Treibmittel, welches bei Erwärmung aufgrund der Bestrahlung mit dem Laserlicht ein das Matrixmaterial schäumendes Gas erzeugt. Aufgrund des von dem Treibmittel erzeugten Gases kommt es bei einer entsprechenden Bestrahlung mit Laserlicht zu einem lokalen Aufschäumen des Kunststoff-Matrixmarerials.

In der US 3 853 783 A1 werden Laserschutzfilter aus Kunststoff umfassend Sulfonamide beschrieben. Die Sulfonamid-Bestandteile weisen eine Absorptionswirkung für eine auftreffende Laserstrahlung im Wellenlängenbereich von 620 bis 720 nm auf. Außerdem haben diese Sulfonamid-Bestandteile stabile Eigenschaften.

Eine Aufgabe der Erfindung besteht deshalb darin, ein Laserschutzmaterial der eingangs bezeichneten Art anzugeben, das eine höhere Laserschutzstufe als bei bislang bekannten Laserschutzmaterialen ermöglicht.

Zur Lösung dieser Aufgabe wird ein Laserschutzmaterial entsprechend den Merkmalen des Patentanspruchs 1 angegeben. Das erfindungsgemäße Laserschutzmaterial umfasst ein im wirksamen Wellenlängenbereich transparentes Matrixmaterial aus einem Kunststoff und ein im Wesentlichen homogen in das Matrixmaterial eingebettetes Absorbermaterial, das eine Laserstrahlung in dem wirksamen Wellenlängenbereich absorbiert und das seine Absorptionswirkung in dem wirksamen Wellenlängenbereich bei einer Erhitzung über eine Grenztemperatur verliert, wobei der Kunststoff des Matrixmaterials bei einer Erhitzung über die Grenztemperatur aufschäumt.

Im Rahmen der Erfindung wurde erkannt, dass sich ein derartiges Laserschutzmaterial mit Vorteil für ein Laserschutzfilter einsetzen lässt. Der wirksame Wellenlängenbereich kann auch als Schutz-Wellenlängenbereich verstanden werden. Bei einer Wellenlänge aus diesem Bereich ist die Filter- und/oder Schutzwirkung gegenüber einer auftreffenden Laserstrahlung gegeben. Mit dem erfindungsgemäßen Laserschutzmaterial lässt sich innerhalb des spezifizierten wirksamen Wellenlängenbereichs eine sehr hohe Laserschutzstufe gemäß EN 207:2009 von insbesondere mindestens D LB7 und ggf. sogar von bis zu D LB8 erreichen. Diese hohe Schutzfunktion hat verschiedene Ursachen.

Ein wichtiger Aspekt ist das in das Matrixmaterial eingebettete oder eingemischte oder im Matrixmaterial gelöste Absorbermaterial, das insbesondere temperaturempfindlich ist und bei dem es sich vorzugsweise um einen Farbstoff handelt. Dieses bevorzugt thermisch instabile Material absorbiert die auftreffende Laserstrahlung, allerdings nur, solange die Temperatur unter einem Grenzwert bleibt. Bei einer Erhitzung über diese Grenztemperatur verliert das Absorbermaterial seine absorbierende Wirkung, da das Absorbermaterial oberhalb dieser Grenztemperatur insbesondere ausbleicht und/oder sich chemisch zersetzt. Dieses Verhalten ist auf den ersten Blick ungünstig, da nach dem Ausbleichen bzw. der chemischen Zersetzung des Absorbermaterials weiterhin auftreffende Laserstrahlung dann in das Laserschutzmaterial eindringt, ohne von dem Absorbermaterial absorbiert zu werden. Im Rahmen der Erfindung wurde aber erkannt, dass gerade dieses vermeintlich ungünstige Verhalten vorteilhaft mit den Eigenschaften des Matrixmaterials kombiniert werden kann, um dann im Endergebnis eine doch erheblich höhere Laserschutzstufe als bisher üblich zu erreichen.

Durch die Absorptionswirkung des Absorbermaterials wird nämlich bis zu dessen Zersetzung die Temperatur im Kunststoff des Matrixmaterials erhöht. Oberhalb der Grenztemperatur erfolgt im Matrixmaterial ein lokales Aufschäumen, welches insbesondere durch eine erwärmungsbedingte chemische Zersetzung des Matrixmaterials zumindest mit verursacht wird. In einem bei diesem Aufschäumvorgang sich insbesondere bildenden Schaumauswurf ist das darin enthaltene Absorbermaterial - wie bereits erläutert - aufgrund der Hitzeeinwirkung nicht mehr absorbierend. Vielmehr ist der Schaumauswurf im Wesentlichen für die weiterhin auftreffende Laserstrahlung transparent. Allerdings wird die Laserstrahlung durch die Struktur des aufgeschäumten Kunststoffs des Matrixmaterials nun sehr stark gestreut, wodurch die Fläche, auf die die Laserstrahlung einwirkt, vergrößert wird, sodass die Leistungsdichte (= Leistung pro Fläche) abnimmt und die weitere Zerstörung des Matrixmaterials und damit auch des Laserschutzmaterials zum Stoppen kommt.

Das Absorbermaterial dient also zu einer anfänglichen Erhitzung des Matrixmaterials, wodurch es im Bereich der auftreffenden Laserstrahlung zu einem lokalen Aufschäumen und als Folge davon zu einer Streuung der auftreffenden Laserstrahlung und damit zu einer Reduzierung der gefährlich hohen Laserleistungsdichte kommt. Aufgrund des Ineinandergreifens der beschriebenen Effekte lässt sich die Schutzwirkung des Laserschutzmaterials erheblich verbessern, sodass verglichen mit dem Stand der Technik höhere Laserschutzstufen möglich sind.

Ein weiterer Vorteil besteht darin, dass bei einem Einsatz des Laserschutzmaterials ein Störfall anhand des Schaumauswurfs, der sich am Ort des Auftreffens der Laserstrahlung gebildet hat, leicht zu erkennen ist. Damit können Gegenmaßnahmen eingeleitet werden, wie zum Beispiel eine Beseitigung des Fehlers in der Einrichtung, in der die Laserstrahlung zum Einsatz gekommen ist und die zum Schutz vor dieser Laserstrahlung mit dem Laserschutzmaterial umgeben ist, oder auch - falls erforderlich - ein Austausch des zu stark beschädigten und damit bei einem erneuten Laserbeschuss nicht mehr sicheren Laserschutzmaterials.

Da das Matrixmaterial ein intrinsisches Aufschäumverhalten aufweist, ist für das günstige Aufschäumen des Matrixmaterials insbesondere kein gesondertes Treibmittel erforderlich. Dadurch reduzieren sich der Herstellungsaufwand und die Herstellungskosten.

Vorteilhafte Ausgestaltungen des erfindungsgemäßen Laserschutzmaterials ergeben sich aus den Merkmalen der von Anspruch 1 abhängigen Ansprüche.

Gemäß einer günstigen Ausgestaltung ist das Matrixmaterial ein duroplastisches Polyurethan. Dieses Kunststoffmaterial ist ein bekannter und auf dem Markt gut verfügbarer Werkstoff, der außerdem auch das erfindungsgemäß vorgesehene Aufschäumverhalten bei einer Erhitzung über eine bestimmte Grenztemperatur aufweist.

Gemäß einer weiteren günstigen Ausgestaltung ist das Polyurethan ein mittels eines RIM-Verfahrens hergestellter Kunststoff. Dieses Verfahren findet bei niedrigen Temperaturen, beispielsweise sogar bei Raumtemperatur statt. Durch die bei der Polymerisierung der Monomere entstehende Reaktionswärme erwärmt sich das Matrixmaterial auf höchstens etwa 100°C. Jedenfalls bleibt die Temperatur aber unterhalb der Grenztemperatur, ab der die Zersetzung des Absorbermaterials und/oder das Aufschäumen des Matrixmaterials beginnt. Mittels des RIM-Verfahrens lassen sich sehr gut duroplastische Polyurethan-Formteile, wie zum Beispiel ein Laserschutzfilter mit in weiten Teilen frei bestimmbarer Formgebung, herstellen.

Gemäß einer weiteren günstigen Ausgestaltung ist das Polyurethan aus Polyol und aus Isocyanat in einem wählbaren Mischungsverhältnis hergestellt. Dies ist günstig, da sich durch das Mischungsverhältnis die mechanische Festigkeit vorgeben lässt. So können insbesondere die mechanische Härte und die mechanische Elastizität der mit dem Laserschutzmaterial hergestellten Laserschutzkomponente eingestellt werden. Hierdurch ist die Fertigung von aufrollbaren Folien ebenso möglich wie von mechanisch stabilen Scheiben zum Einsatz als Laserschutzfilter in Laserschutzbrillen oder Laserschutzfenstern.

Gemäß einer weiteren günstigen Ausgestaltung ist das Absorbermaterial entweder in das Polyol oder in das Isocyanat im Wesentlichen homogen eingemischt. Dadurch, dass die Zumischung des Absorbermaterials bereits in eine der Monomer-Komponenten erfolgt, erreicht man eine besonders gleichmäßige Einbettung des Absorbermaterials in das letztendlich resultierende Polyurethan-Matrixmaterial.

Gemäß einer weiteren günstigen Ausgestaltung ist das Absorbermaterial in das Polyol eingemischt. Dies erleichtert die Handhabung, da eine grundsätzlich ebenfalls mögliche Zumischung in das Isocyanat aufgrund der hochgiftigen Eigenschaft dieses Monomers zu einem deutlich aufwändigeren Herstellungsverfahren führen würde.

Gemäß einer weiteren günstigen Ausgestaltung ist das Absorbermaterial ein Farbstoff aus oder mit Amminium oder aus oder mit einem Cyanin oder aus oder mit einem Schwermetall-Komplex. Diese Stoffe lassen sich einfach in das Kunststoff-Matrixmaterial einmischen. Außerdem weisen sie die erfindungsgemäß vorgesehene Eigenschaft des Verlustes der absorbierenden Wirkung bei einer Erhitzung über eine Grenztemperatur auf. Bei dem Schwermetall-Komplex kann es sich insbesondere um einen Nickel-Basiskomplex handeln.

Gemäß einer weiteren günstigen Ausgestaltung ist das Absorbermaterial mit einer wählbaren Konzentration in das Matrixmaterial eingebettet. Dadurch lassen sich das Absorptionsverhalten und insbesondere auch der Volumenbereich, in dem die Wechselwirkung mit der Laserstrahlung stattfindet, einstellen. Somit kann zumindest in gewissem Umfang vorzugsweise auch eingestellt werden, ab welchen Leistungs- oder Energiedichten dieser Wechselwirkungsmechanismus startet.

Gemäß einer weiteren günstigen Ausgestaltung ist für die Grenztemperatur ein Wert von mindestens 100°C, insbesondere von mindestens 150°C und bevorzugt von mindestens 200°C vorgesehen. Die Grenztemperatur liegt dann vorzugsweise immer noch über der Herstellungstemperatur während der Herstellung der aus dem Laserschutzmaterial gefertigten Laserschutzkomponente, sodass der Verlust der absorbierenden Wirkung im Absorbermaterial und das Aufschäumen im Matrixmaterial erst später, nämlich während eines Störfalls beim Auftreffen einer energiereichen Laserstrahlung, und nicht schon während der Herstellung auftreten.

Gemäß einer weiteren günstigen Ausgestaltung liegt der wirksame Wellenlängenbereich zwischen 750 nm und 1150 nm, und damit insbesondere im nahen Infrarot-Wellenlängenbereich. In diesem Wellenlängenbereich liegen einige sehr leistungsstarke Laseranwendungen, sodass gerade dort Laserschutzmaterialien mit einer besonders hohen Laserschutzstufe von Vorteil sind.

Gemäß einer weiteren günstigen Ausgestaltung ist das Matrixmaterial außer im wirksamen Wellenlängenbereich auch in einem weiteren Wellenlängenbereich transparent. Dies ist insbesondere bei einer Anwendung für ein Laserschutzfilter einer Laserschutzbrille oder einer Laserschutzscheibe von Vorteil. Diese Laserschutzkomponenten sollen nämlich im sichtbaren Wellenlängenbereich transparent sein, damit das Bedienpersonal durch den Laserschutzfilter hindurchblicken kann. Daneben ist natürlich auch eine Transparenz in dem wirksamen Wellenlängenbereich, der sich aber durchaus von dem sichtbaren Wellenlängenbereich unterscheiden kann, erforderlich. Grundsätzlich gibt es aber auch andere Anwendungsfälle, bei denen die Transparenz nur in dem wirksamen Wellenlängenbereich vorgesehen ist. Beispiele für derartige andere Anwendungen sind Laserschutzwände, Laserschutzvorhänge, aber auch Fassungen für Laserschutzbrillen oder Beschichtungen für Fassungen von Laserschutzbrillen.

Eine weitere Aufgabe der Erfindung besteht darin, eine Laserschutzkomponente mit einer gegenüber bislang bekannten Laserschutzkomponenten verbesserten Laserschutzstufe anzugeben.

Zur Lösung dieser Aufgabe wird eine Laserschutzkomponente entsprechend den Merkmalen des Patentanspruchs 12 angegeben. Die erfindungsgemäße Laserschutzkomponente umfasst ein Element aus dem vorstehend beschriebenen erfindungsgemäßen Laserschutzmaterial oder dessen ebenfalls vorstehend beschriebenen vorteilhaften Ausgestaltungen. Das erfindungsgemäße Laserschutzmaterial und seine vorteilhaften Ausgestaltungen werden also insbesondere zur Herstellung einer Laserschutzkomponente verwendet. Eine solche Laserschutzkomponente und ihre Ausgestaltungen bieten demnach im Wesentlichen die gleichen Vorteile, die bereits im Zusammenhang mit dem erfindungsgemäßen Laserschutzmaterial und dessen Ausgestaltungen beschrieben worden sind.

Vorteilhafte Ausgestaltungen der Laserschutzkomponente ergeben sich aus den vom Anspruch 12 abhängigen Ansprüchen. Gemäß einer günstigen Ausgestaltung ist das Element der Laserschutzkomponente ein Laserschutzfilter einer Laserschutzbrille oder einer Laserschutzscheibe oder ein Bestandteil einer Laserschutzwand oder eines Laserschutzvorhangs. Ebenso kann das Element die Fassung oder Teil der Fassung einer Laserschutzbrille oder einer Laserschutzscheibe sein. In diesem Zusammenhang ist ein Laserschutzfilter nicht nur als beispielsweise im sichtbaren Wellenlängenbereich transparente Scheibe, sondern, wie in den einschlägigen Normungsvorschriften angegeben, allgemeiner als ein Element zu verstehen, das Laserstrahlung in dem spezifizierten wirksamen Wellenlängenbereich abhält und nicht passieren lässt. Insofern sind auch Laserschutzwände und Laserschutzvorhänge als derartige Laserschutzfilter zu verstehen. Jedenfalls lässt sich das Laserschutzmaterial mit Vorteil für die genannten Laserschutzkomponenten einsetzen. Es resultiert eine sehr hohe Laserschutzstufe.

Weitere Merkmale, Vorteile und Einzelheiten der Erfindung ergeben sich aus der nachfolgenden Beschreibung eines Ausführungsbeispiels anhand der Zeichnung. Es zeigt:
- Fig. 1: ein Ausführungsbeispiel einer Laserschutzkomponente aus einem aufschäumenden Laserschutzmaterial in einer Querschnittsdarstellung, und
- Fig. 2 und 3: die Laserschutzkomponente gemäß Fig. 1 beim Auftreffen einer Laserstrahlung.

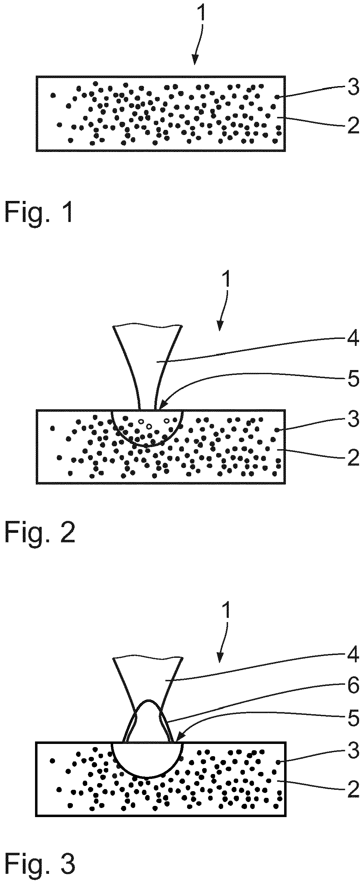

Einander entsprechende Teile sind in Fig. 1 bis 3 mit denselben Bezugszeichen versehen. Auch Einzelheiten des im Folgenden näher erläuterten Ausführungsbeispiels können für sich genommen eine Erfindung darstellen oder Teil eines Erfindungsgegenstands sein.

In Fig. 1 ist ein Ausführungsbeispiel einer Laserschutzkomponente 1 in Form einer Scheibe oder eines Filters einer Laserschutzbrille in Querschnittsdarstellung abgebildet. Die Laserschutzkomponente 1 ist aus einem Laserschutzmaterial hergestellt, das ein Matrixmaterial 2 umfasst, in das ein Absorbermaterial 3 weitgehend homogen eingebettet ist.

Bei dem Matrixmaterial 2 des Ausführungsbeispiels handelt es sich um ein duroplastisches Polyurethan. Das Absorbermaterial 3 ist bei dem gezeigten Ausführungsbeispiel ein Farbstoff auf Amminium-Basis.

Die Laserschutzkomponente 1 ist für den nahen infraroten Wellenlängenbereich spezifiziert. Laserstrahlung 4 mit einer Wellenlänge zwischen 750 nm und 1150 nm wird am Durchtritt durch die Laserschutzkomponente 1 gehindert. Die Laserschutzkomponente weist eine Laserschutzstufe gemäß EN 207:2009 von mindestens D LB7 auf.

Das Matrixmaterial 2 des Laserschutzmaterials ist in dem genannten wirksamen Wellenlängenbereich für die Laserstrahlung 4 transparent. Dagegen absorbiert das Absorbermaterial 3 die Laserstrahlung 4 in dem wirksamen Laserbereich, zumindest insbesondere solange die Temperatur unter einer Grenztemperatur liegt.

Die Wirkungsweise der Laserschutzkomponente 1 bei Auftreffen einer Laserstrahlung 4 wird im Folgenden anhand von Fig. 2 und 3 erläutert.

In dem Bereich 5, in dem die Laserstrahlung 4 auf die Laserschutzkomponente 1 auftrifft, absorbiert das Absorbermaterial 3 zunächst die Energie der Laserstrahlung 4. Dadurch kommt es in diesem Bereich 5 zu einer lokalen Erwärmung. Steigt die Temperatur aufgrund dessen über eine Grenztemperatur, die beim Ausführungsbeispiel bei etwa 260°C liegt, bleicht der Farbstoff des Absorbermaterials 3 aus und verliert seine absorbierende Wirkung. Danach ist er für die Laserstrahlung 4 in dem wirksamen Wellenlängenbereich ebenso transparent wie das Matrixmaterial 2.

Außerdem führt die lokale Erwärmung dazu, dass sich das Polyurethan des Matrixmaterials 2, welches für die Laserstrahlung 4 in dem wirksamen Wellenlängenbereich transparent ist, sich chemisch zersetzt und aufschäumt. Nach kurzer Zeit kommt es zu einem Schaumauswurf 6, welcher für die Laserstrahlung 4 transparent ist, zum einen aufgrund der ohnehin gegebenen Transparenz des Matrixmaterials 2 und zum anderen aufgrund des nicht mehr absorbierenden Absorbermaterials 3. Die weiterhin auftreffende Laserstrahlung 4 wird in diesem Bereich 5 und dem Schaumauswurf 6 also nicht mehr absorbiert. Stattdessen kommt es aufgrund der schaumartigen Struktur zu einer starken Streuung der Laserstrahlung 4. Dadurch wird die Fläche, auf die die Laserstrahlung 4 einwirkt, vergrößert, sodass die Leistungsdichte (Leistung pro Fläche) abnimmt und die weitere Zerstörung des Laserschutzmaterials der Laserschutzkomponente 1 stoppt.

Aufgrund der vorteilhaften temperaturabhängigen absorbierenden Wirkung des Absorbermaterials 3, dessen Farbstoff thermisch instabil ist und bei Überschreiten der Grenztemperatur sein absorbierendes Verhalten verliert, sowie der zugleich mit einer Temperaturerhöhung über die Grenztemperatur einhergehenden Aufschäumung des Matrixmaterials 2 aus duroplastischem Polyurethan resultiert also eine extrem effiziente Schutzwirkung auch gegenüber einer sehr leistungsstarken auftreffenden Laserstrahlung 4. Insgesamt ergibt sich so die genannte Laserschutzstufe von mindestens D LB7, die mit anderen bislang zum Schutz vor Laserstrahlung gebräuchlichen Materialen nicht erreicht werden konnte.

## Patentansprüche

1. Laserschutzmaterial für ein Laserschutzfilter (1) mit einem wirksamen Wellenlängenbereich, bestehend aus:
a) einem im wirksamen Wellenlängenbereich transparentes Matrixmaterial (2) aus einem Kunststoff, und
b) einem im Wesentlichen homogen in das Matrixmaterial (2) eingebettetes Absorbermaterial (3), das eine Laserstrahlung (4) in dem wirksamen Wellenlängenbereich absorbiert und das seine Absorptionswirkung in dem wirksamen Wellenlängenbereich bei einer Erhitzung über eine Grenztemperatur verliert, wobei
c) der Kunststoff des Matrixmaterials (2) bei einer Erhitzung über die Grenztemperatur aufschäumt, und
d) das Matrixmaterial (2) ein intrinsisches Aufschäumverhalten aufweist.

2. Laserschutzmaterial nach Anspruch 1, **dadurch gekennzeichnet, dass** das Matrixmaterial (2) ein duroplastisches Polyurethan ist.

3. Laserschutzmaterial nach Anspruch 2, **dadurch gekennzeichnet, dass** das Polyurethan mittels eines RIM-Verfahrens hergestellter Kunststoff ist.

4. Laserschutzmaterial nach Anspruch 2 oder 3, **dadurch gekennzeichnet, dass** das Polyurethan aus Polyol und aus Isocyanat in einem wählbaren Mischungsverhältnis hergestellt ist.

5. Laserschutzmaterial nach Anspruch 4, **dadurch gekennzeichnet, dass** das Absorbermaterial (3) entweder in das Polyol oder in das Isocyanat im Wesentlichen homogen eingemischt ist.

6. Laserschutzmaterial nach Anspruch 5, **dadurch gekennzeichnet, dass** das Absorbermaterial (3) in das Polyol eingemischt ist.

7. Laserschutzmaterial nach einem der vorhergehenden Ansprüche, **dadurch gekennzeichnet, dass** das Absorbermaterial (3) ein Farbstoff aus oder mit Amminium oder aus oder mit einem Cyanin oder aus oder mit einem Schwermetall-Komplex ist.

8. Laserschutzmaterial nach einem der vorhergehenden Ansprüche, **dadurch gekennzeichnet, dass** das Absorbermaterial (3) mit einer wählbaren Konzentration in das Matrixmaterial eingebettet ist.

9. Laserschutzmaterial nach einem der vorhergehenden Ansprüche, **dadurch gekennzeichnet, dass** für die Grenztemperatur ein Wert von mindestens 100°C, insbesondere von mindestens 150°C und bevorzugt von mindestens 200°C vorgesehen ist.

10. Laserschutzmaterial nach einem der vorhergehenden Ansprüche, **dadurch gekennzeichnet, dass** der wirksame Wellenlängenbereich zwischen 750 nm und 1150 nm liegt.

11. Laserschutzmaterial nach einem der vorhergehenden Ansprüche, **dadurch gekennzeichnet, dass** das Matrixmaterial (2) außer im wirk samen Wellenlängenbereich auch in einen weiteren Wellenlängenbereich transparent ist.

12. Laserschutzkomponente umfassend ein Element aus dem Laserschutzmaterial nach einem der vorhergehenden Ansprüche.

13. Laserschutzkomponente nach Anspruch 12, **dadurch gekennzeichnet, dass** das Element ein Laserschutzfilter (1) einer Laserschutzbrille oder einer Laserschutzscheibe oder ein Bestandteil einer Laserschutzwand oder eines Laserschutzvorhangs ist.

## Claims

1. Laser protection material for a laser protection filter (1) having an effective wavelength range, the laser protection material consisting of
a) a matrix material (2) of a plastic which is transparent in the effective wavelength range, and
b) an absorber material (3) which is substantially homogeneously embedded into the matrix material (2), wherein the absorber material (3) absorbs a laser radiation (4) in the effective wavelength range and loses its absorptive capacity in the effective wavelength range when heated to a temperature above a threshold temperature, wherein
c) the plastic of the matrix material (2) starts to foam when heated to a temperature above the threshold temperature, and
d) the matrix material (2) has an intrinsic foaming behaviour.

2. Laser protection material according to claim 1, **characterised in that** the matrix material (2) is a thermosetting polyurethane.

3. Laser protection material according to claim 2, **characterised in that** the polyurethane is a plastic produced according to a RIM method.

4. Laser protection material according to claim 2 or 3, **characterised in that** the polyurethane is produced from polyol and from isocyanate in a selectable mixing ratio.

5. Laser protection material according to claim 4, **characterised in that** the absorber material (3) is embedded either into the polyol or into the isocyanate in a substantially homogeneous manner.

6. Laser protection material according to claim 5, **characterised in that** the absorber material (3) is blended into the polyol.

7. Laser protection material according to any one of the preceding claims, **characterised in that** the absorber material (3) is a colorant from or including amminium or from or including a cyanine or from or including a heavy metal complex.

8. Laser protection material according to any one of the preceding claims, **characterised in that** the absorber material (3) is embedded into the matrix material in a selectable concentration.

9. Laser protection material according to any one of the preceding claims, **characterised in that** a value of at least 100°C, in particular of at least 150°C, and preferably of at least 200°C is defined as threshold temperature.

10. Laser protection material according to any one of the preceding claims, **characterised in that** the effective wavelength range is between 750 nm and 1150 nm.

11. Laser protection material according to any one of the preceding claims, **characterised in that** the matrix material (2) is transparent in another wavelength range differing from the effective wavelength range.

12. Laser protection component comprising an element made of the laser protection material according to any one of the preceding claims.

13. Laser protection component according to claim 12, **characterised in that** the element is a laser protection filter (1) of a pair of laser protection goggles or of a laser protection screen or a component of a laser protection wall or of a laser protection curtain.

## Revendications

1. Matériau de protection laser pour un filtre de protection laser (1) pourvu d'une plage de longueurs d'ondes efficace, constitué de :
a) un matériau de matrice (2) transparent dans la plage de longueurs d'ondes efficace, composé d'un plastique, et
b) un matériau absorbant (3) incorporé dans le matériau de matrice (2) de manière essentiellement homogène, qui absorbe un rayon laser (4) dans la plage de longueurs d'ondes efficace et qui perd son action absorbante dans la plage de longueurs d'ondes efficace lors d'un chauffage à une température limite, dans lequel
c) le plastique du matériau de matrice (2) mousse à un chauffage au-delà de la température limite, et
d) le matériau de matrice (2) présente un comportement de moussage intrinsèque.

2. Matériau de protection laser selon la revendication 1, **caractérisé en ce que** le matériau de matrice (2) est un polyuréthane duroplastique.

3. Matériau de protection laser selon la revendication 2, **caractérisé en ce que** le polyuréthane est un plastique fabriqué par le biais d'un procédé RIM.

4. Matériau de protection laser selon la revendication 2 ou 3, **caractérisé en ce que** le polyuréthane est fabriqué à partir de polyol et d'isocyanate en un rapport de mélange sélectionnable.

5. Matériau de protection laser selon la revendication 4, **caractérisé en ce que** le matériau absorbant (3) est incorporé de manière essentiellement homogène dans le polyol ou dans l'isocyanate.

6. Matériau de protection laser selon la revendication 5, **caractérisé en ce que** le matériau absorbant (3) est incorporé dans le polyol.

7. Matériau de protection laser selon l'une des revendications précédentes, **caractérisé en ce que** le matériau absorbant (3) est un colorant constitué ou comprenant de l'amminium ou constitué ou comprenant une cyanine ou constitué ou comprenant un complexe de métaux lourds.

8. Matériau de protection laser selon l'une des revendications précédentes, **caractérisé en ce que** le matériau absorbant (3) est incorporé dans le matériau de matrice à une concentration sélectionnable.

9. Matériau de protection laser selon l'une des revendications précédentes, **caractérisé en ce qu'**une valeur d'au moins 100 °C, en particulier d'au moins 150 °C, et de préférence d'au moins 200 °C, est prévue pour la température limite.

10. Matériau de protection laser selon l'une des revendications précédentes, **caractérisé en ce que** la plage de longueurs d'ondes efficace est comprise entre 750 nm et 1150 nm.

11. Matériau de protection laser selon l'une des revendications précédentes, **caractérisé en ce que** le matériau de matrice (2) est transparent dans une autre plage de longueurs d'ondes en dehors de la plage de longueurs d'ondes efficace.

12. Composant de protection laser comprenant un élément constitué du matériau de protection laser selon l'une des revendications précédentes.

13. Composant de protection laser selon la revendication 12, **caractérisé en ce que** l'élément est un filtre de protection laser (1) d'une paire de lunettes de protection laser ou d'une vitre de protection laser ou un ingrédient d'une paroi de protection laser ou d'un rideau de protection laser.
